# EUROPEAN PATENT SPECIFICATION

(11) **EP 0 521 310 B1**
(45) Date of publication and mention of the grant of the patent: **20.01.1999**
(21) Application number: 92109459.5
(22) Date of filing: 04.06.1992
(51) Int. Cl.: A61K 31/44, A61K 9/20

(54) **Solid dispersion tablets containing 1,4-dihydropyridine derivatives and process for the preparation thereof**
1,4-Dihydropyridin-Derivate enthaltende Tabletten des Typs Feststoffdispersion und Verfahren zu deren Herstellung
Comprimés de type dispersion solide contenant des dérivés de 1,4-dihydropyridine et procédé pour leur préparation

(30) Priority: 05.06.1991 JP 134344/91
(43) Date of publication of application: 07.01.1993
(73) Proprietor: Fujirebio Inc., Tokyo (JP); Ajinomoto Co., Inc., Tokyo 104 (JP)
(72) Inventor: Watanabe, Daiichi, B-1302, Matsudo-shi, Chiba-ken (JP); Mimachi, Hiroko, Meguro-ku, Tokyo (JP)
(74) Representative: Hansen, Bernd, Dr. Dipl.-Chem.

(56) References cited:
- EP-A- 0 137 198
- EP-A- 0 371 471
- DE-A- 2 822 882
- DATABASE WPI Week 8332, Derwent Publications Ltd., London, GB; AN 83-731210 & JP-A-58 109 411 (SHIONOGI KK) 29 June 1983
- CHEMICAL ABSTRACTS, vol. 101, no. 18, 29 October 1984, Columbus, Ohio, US; abstract no. 157544g, A. HASEGAWA ET AL 'Solid dispersion obtained from nifedipine and an enteric coating agent. I. Dissolution behavior'
- JOURNAL OF PHARMACEUTICAL SCIENCES, vol.60, no.9, September 1971 pages 1281 - 1302 WIN LOUNG CHIOU ET AL 'Pharmaceutical Applications of Solid Dispersion Systems'

## Description

### Background of the Invention

### Field of the Invention

The present invention relates to a new solid dispersion (SD) tablet containing a substantially water-insoluble 1,4-dihydropyridine derivative as a pharmaceutical active agent, and to a process for the preparation of the SD tablets.

### Prior Art

Some drugs or pharmaceutically active agents have poor absorption through mammalian digestive tract because of their very slight solublity in water, in spite of their excellent pharmacological effects. In general, such drugs can not be practically used or must be administered in a large amount. The 1,4-dihydropyridine derivative having a vasodilating activity is one of them, and this compound is so substantially insoluble in water that it, when administered orally, is quite poorly absorbed in vivo, thus resulting in a practical problem that the bioavailability thereof is low.

For example, Nifedipine (1,4-dihydro-2,6-dimethyl-4-(o-nitrophenyl)-3,5-pyridinedicarboxylic acid dimethyl ester; see US Patent No. 3,644,627) has been normaly used as a drug for the treatment of an attack of angina pectoris, but it has the disadvantage that in vivo absorption is slow, and therefore various rapid absorption type (or fast acting) oral-Nifedipine preparations have been proposed. Examples of such preparations are the solid which was prepared by dissolving the mixture of Nifedipine and polyvinyl pyrrolidone in an organic solvent followed by removal of the solvent by evaporation (Japanese Patent Application Laying Open (KOKAI) No. 54-2316), and the soft capsule in which the solution of Nifedipine in liquid polyethylene glycol was filled (Japanese Patent Application Laying Open (KOKAI) No. 48-28621).

On the other hand, when Nifedipine is used as an antihypertensive agent, the duration of its effect is required rather than its fast action. In this case, slow absorption type of Nifedipine preparations are desired. An example of such Nifedipine preparations includes the mixed granules of Nifedipine-containing granules with the granules which were obtained by forming the coating consisting of substantially water-insoluble material and an enteric high molecular material onto the surface of the Nifedipine-containing granules (Japanese Patent Application Laying Open (KOKAI) No. 58-46019).

With respect to the traditional slow absorption type of Nifedipine preparations, it has been required that their levels in blood are maintained at a constant effective level for some time.

The problem tackled by the present invention is to develop a medicinal preparation which can maintain good inherent in vivo absorption of its active drug i.e. the in vivo absorption characteristics possessed by traditional non-sustained release preparations, but which simultaneously improve the absorption rate in vivo. Such a medicinal preparation would be more ideal in view of that it can remove drawbacks such as side effects caused in association with the maintenance of the blood level of an active ingredient.

The object of the present invention is to provide a pharmaceutical solid dispersion (SD) tablet containing a substantially water-insoluble 1,4-dihydropyridine derivative as a pharmaceutical active agent which improves an absorption rate thereof while retaining the desirable in vivo absorption as described above.

### Summary of the Invention

The present invention relates to a pharmaceutical solid dispersion (SD) tablet comprising a solid dispersion material (a) which comprises a substantially water-insoluble 1,4-dihydropyridine derivative in amorphous form as a pharmaceutical active agent represented by the general formula [I]: 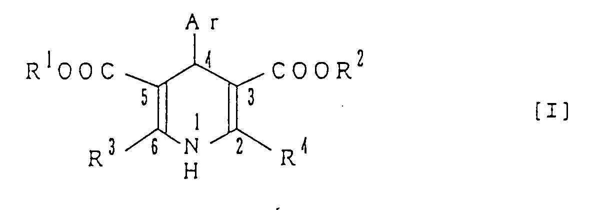 wherein Ar is a substituted aryl or benzofurazanyl group, R¹ is a substituted or unsubstituted alkyl group, R² is a substituted or unsubstituted alkyl or alkenyl group or a substituted piperidyl group, R³ and R⁴ each independently represent a substituted or unsubstituted alkyl group, and the substituent R¹OCO- at the 5-position may be replaced by a dialkylphosphonoate or alkylene dioxyphosphoryl group, or a pharmaceutically acceptable acid addition salt thereof and hydroxypropyl cellulose; and a solid dispersion material (b) which comprises said 1,4-dihydropyridine derivative or its pharmaceutically acceptable acid addition salt and hydroxypropyl methyl cellulose phthalate.

The SD tablet of the present invention improves the bioavailability of the active agent without altering the inherent in vivo absorption of the agent

### Brief Description of the Drawings

Fig. 1 shows time course of the blood level of FRC8653 when a wet granule-compression tablet (Comparative Example 1) was orally administered to a dog (10 mg of FRC8653/body, n=6).
Fig. 2 shows dissolution profile of the HPC/HP55 (3/7) SD tablet (Comparative Example 2) prepared by mixing FRC8653, HPC and HP55 simultaneously, as compared with the HPC/HP55 mixed (3/7) SD tablet of the present invention.
Fig. 3 shows DSC curves of the solid dispersion system consisting of FRC8653 and HPC, and of FRC8653 alone, where the numbers described mean (1): FRC8653/HPC (1/5); (2): FRC8653/HPC (1/4); (3): FRC8653/HPC (1/3); (4): FRC8653/HPC (1/2); (5): FRC8653/HPC (1/1); and (6): FRC8653.
Fig. 4 shows dissolution profile of FRC8653 from the HPC.SD tablet.
Fig. 5 shows time course of the blood level of FRC8653 when the HPC.SD tablet was orally administered to a dog.
Fig. 6 shows DSC curves of the solid dispersion system consisting of FRC8653 and HP55, and of FRC8653 alone, where the numbers described mean (1): FRC8653/HP55 (1/5); (2): FRC8653/HP55 (1/4); (3): FRC8653/HP55 (1/3); (4): FRC8653/HP55 (1/2); (5): FRC8653/HP55 (1/1); and (6): FRC8653.
Fig. 7 shows dissolution profiles of FRC8653 from the HPC.SD tablet and from the HPC/HP55 mixed SD tablets.
Fig. 8 shows time course of the blood level of FRC8653 when the HPC/HP55 mixed (3/7) SD tablet was orally administered to a dog (10 mg of FRC8653/body, n=6).
Fig. 9 shows dissolution profiles of FRC8653 from the HPC/HP55 mixed (3/7) SD tablets (A in the figure) and from the HPC/HP55 mixed (0/10) SD tablets (B in the figure) at acidic or neutral pH.
Fig. 10 shows the comparison of % dissolution between the HPC/HP55 mixed (3/7) SD tablet and the HPC/HP55 mixed (3/7) SD granules

### Detailed Description of the Invention

The present invention provides a pharmaceutical solid dispersion (SD) tablet comprising a solid dispersion material (a) which comprises a substantially water-insoluble-1,4-dihydropyridine derivative as a pharmaceutical active agent represented by the general formula [I]: 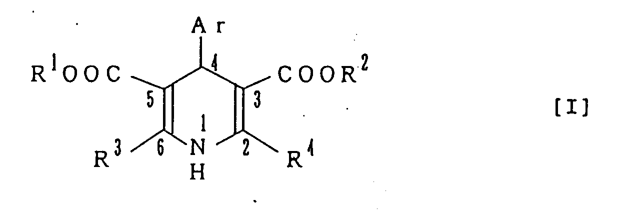 wherein Ar is a substituted aryl or benzofurazanyl group, R¹ is a substituted or unsubstituted alkyl group, R² is a substituted or unsubstituted alkyl or alkenyl group or a substituted piperidyl group, R³ and R⁴ each independently represent a substituted or unsubstituted alkyl group, and the substituent R¹OCO- at the 5-position may be replaced by a dialkylphosphonoate or alkylene dioxyphosphoryl group, or a pharmaceutically acceptable acid addition salt thereof and hydroxypropyl cellulose; and a solid dispersion material (b) which comprises said 1,4-dihydropyridine derivative or its pharmaceutically acceptable acid addition salt and hydroxypropyl methyl cellulose phthalate.

In the SD tablet according to this invention, both water-soluble high molecular hydroxypropyl cellulose and enteric high molecular hydroxypropyl methyl cellulose phthalate are essential as binders (carriers), as well as said 1,4-dihydropyridine derivative as a drug or pharmaceutical active agent.

In the above mentioned general formula [I], examples of the substituted or unsubstituted alkyl group are straight-chain or branched chain alkyl groups with or without a substituent(s) such as dialkylamino, piperadinyl, alkoxy or -ONO₂, preferably lower alkyl groups, more preferably C₁-C₄ alkyl groups; examples of the substituted or unsubstituted alkenyl group are alkenyl groups with or without a substituent(s) such as phenyl, preferably lower alkenyl groups, more preferably C₁-C₄ alkenyl groups; and examples of the substituted aryl group are phenyl groups with a substituent(s) such as halogen, nitro or furazanyl.

Exemplified 1,4-dihydropyridine derivatives include Nifedipine known as a drug for the treatment of angina pectoris and as a drug having vasodilating activity, and its derivatives such as Nisoldipine (isobutyl methyl 1,4-dihydro-2,6-dimethyl-4-(o-nitrophenyl)-3,5-pyridinedicarboxylate), Nicardipine (2-(benzylmethylamino)ethyl methyl 1,4-dihydro-2,6-dimethyl-4-(m-nitrophenyl)-3,5-pyridinedicarboxylate HCl), Nimodipine (isopropyl 2-methoxyethyl 1,4-dihydro-2,6-dimethyl-4-(m-nitrophenyl)-3,5-pyridinecarboxylate), Nitrendipine (ethyl methyl 1,4-dihydro-2,6-dimethyl-4-(m-nitrophenyl)-3,5-pyridinedicarboxylate), Nilvadipine (5-isopropyl-3-methyl-2-cyano-1,4-dihydro-6-methyl-4-(m-nitrophenyl)-3,5-pyridinedicarboxylate), Manidipine (2-[4-(diphenylmethyl)-1-piperazinyl]ethyl methyl (+/-)-1,4-dihydro-2,6-dimethyl-4-(m-nitrophenyl)-3,5-pyridinedicarboxylate dihydrochloride), FRC8653 (2-methoxyethyl (E)-3-phenyl-2-propen-1-yl (+/-)-1,4-dihydro-2,6-dimethyl-4-(3-nitrophenyl)-3,5- pyridinedicarboxylate; Japanese Patent Application Laying Open (KOKAI) No. 60-233058). In addition, said derivatives may be in the form of pharmaceutically acceptable inorganic or organic acid addition salts with for example hydrochloric acid, sulfuric acid, phosphoric acid, citric acid, oxalic acid, fumaric acid, maleic acid, or tartaric acid.

The SD tablet of this invention can be prepared by mixing a solid dispersion material (a) containing 4 parts by weight or more and preferably 4 to 5 parts by weight of hydroxypropyl cellulose based on one part by weight of a 1,4-dihydropyridine derivative or its acid addition salt, with a solid dispersion material (b) containing 2 parts by weight or more and preferably 2 to 5 parts by weight of hydroxypropyl cellulose phthalate based on one part by weight of said 1,4-dihydropyridine derivative or its acid addition salt, and then by compressing the resulting mixture to form solid dispersion tablets. The mixing ratio of the solid dispersion material (a) to the solid dispersion material (b) is selected such that an absorption rate of the active agent is improved without altering the inherent in vivo absorption thereof. The preferred ratio is (a)/(b) = 2.5/7.5 - 3.5/6.5 (weight/weight), more preferably (a)/(b) =3 /7.

The term "solid dispersion material" as used herein means such a solid that a substantially water-insolube 1,4-dihydropyridine derivative represented by the general formula [I] was dispersed in a monomolecular form in an inert carrier (see W. L. Chiou and S. Riegelman, J. Pharm. Sci., 60, 1281 (1971)). In this case, the active agent is present in the carrier in an amorphous form thereof. Whether it is amorphous can be determined by thermal analysis.

The term "substantially water-insoluble" as used herein means that a 1,4-dihydropyridine derivative represented by the general formula [I] is very slightly soluble or practically insoluble in water.

Moreover, the SD tablet of the present invention may preferably contain one or more excipients, disintegrating agents, or lubricants, or combinations thereof in the solid dispersion materials (a) and (b).

Examples of the excipients are lactose, mannitol, sucrose, corn starch, crystalline cellulose, and natural or synthetic alminium silicate and preferably magnesium methasilicate aluminate. When the magnesium methasilicate aluminate is used, it is usually added to the solid dispersion materials (a) or (b), and their amounts added are: 11 parts by weight or more and preferably 11 to 12 parts by weight relative to one part by weight of a pharmaceutical active agent in the solid dispersion material (a); and 5.5 parts by weight or more and preferably 5.5 to 12 parts by weight relative to one parts by weight of the active agent in the solid dispersion material (b).

Examples of the disintegrating agents are gum arabi, sodium alginate, sodium carboxymethyl cellulose, fibrous calcium gluconate, etc, and examples of the lubricants are talc, magnesium stearate, etc.

If desired, the SD tablet of the present invention may be film-coated with a coating agent. Examples of the coating agent are hydroxypropyl methyl cellulose, ethyl cellulose, etc. The coating agent may contain, for example, an opaquing agent such as titanium oxide or a plasticizer such as polyethylene glycol.

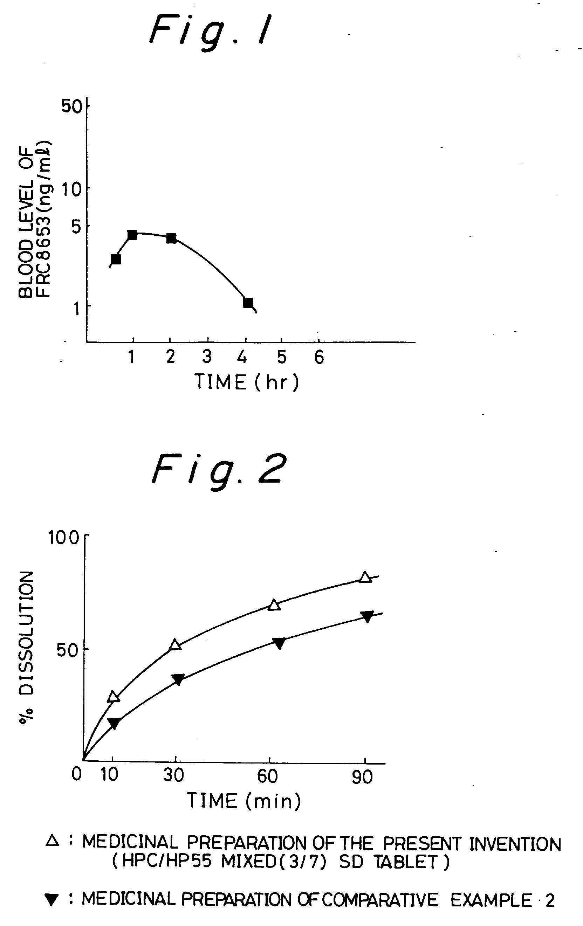

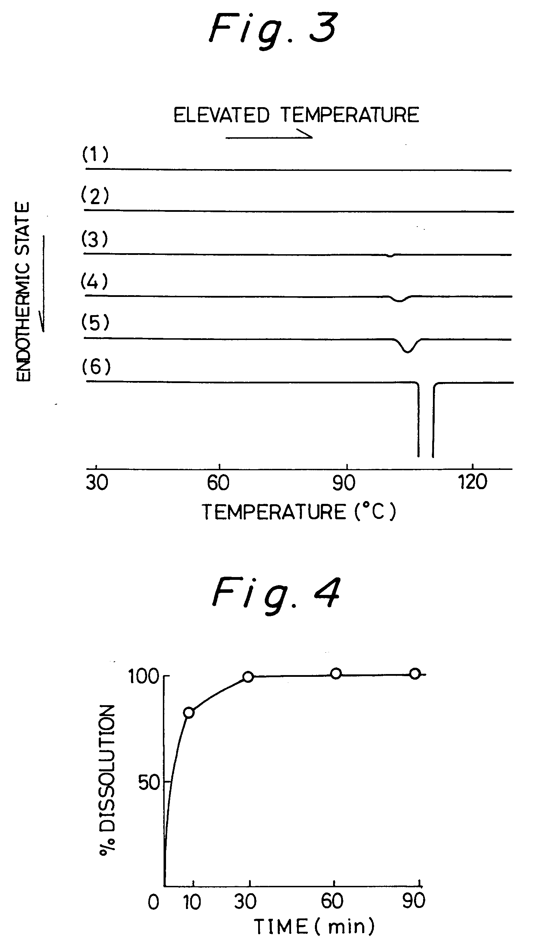

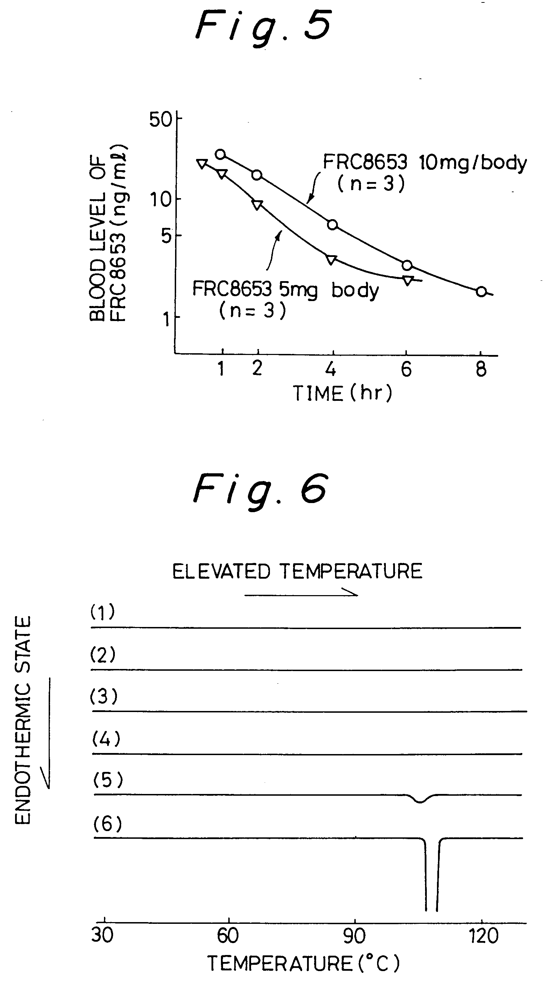

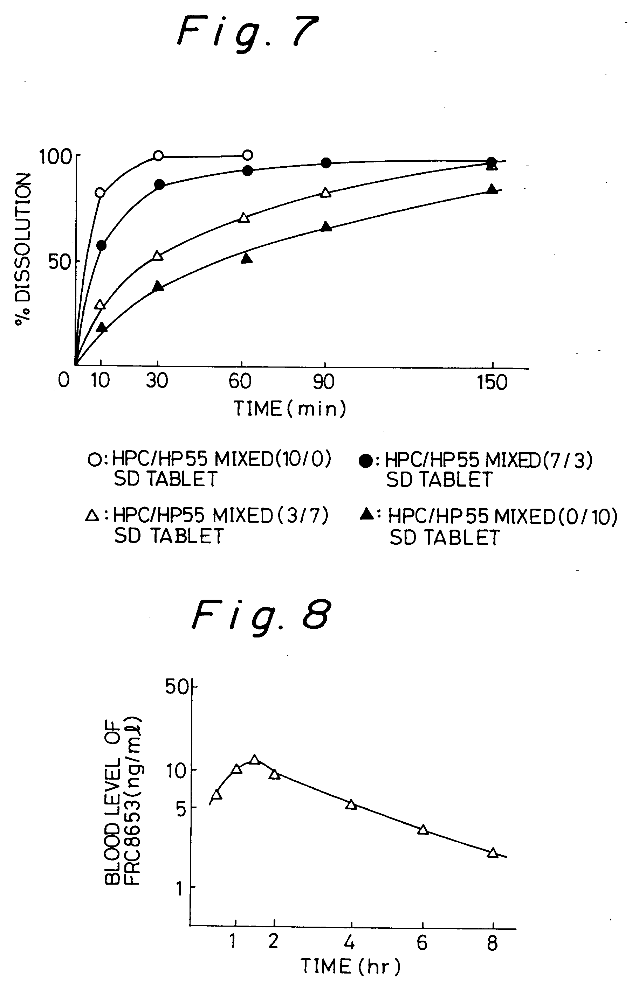

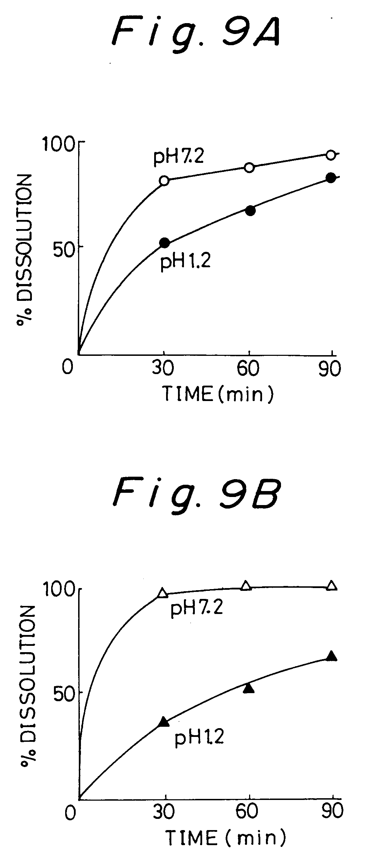

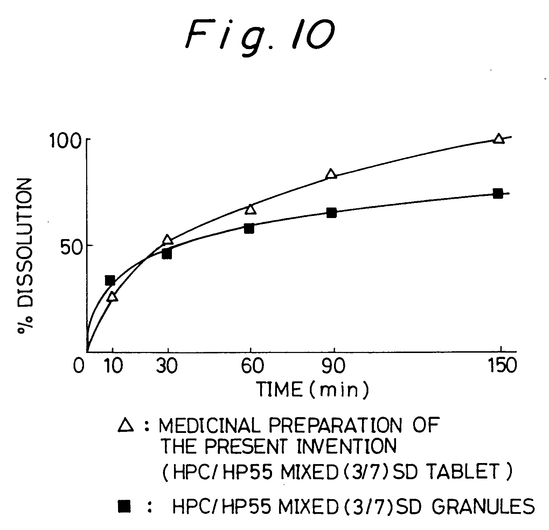

The SD tablet according to the present invention, as elucidated by FRC8653, has some advantages that the active agent is completely dissolved within the stomach without rapidly dissolving over a desired level; the inherent in vivo absorption (Fig. 1) of the active agent is not changed (Fig. 8); and this tablet has as high in vivo absorption rate as the hydroxypropyl cellulose solid dispersion tablet (Example 2), and these advantages are different from those of Nifezipine solid dispersion fine particles (Kanebo Inc., Japan) sold as a medicament for the treatment of angina pectoris, which is a rapid absorption or fast acting type of preparation . The advantages of the present invention are based on the facts that the time required to reach the maximum blood level of a 1,4-dihydropyridine derivative after oral administration (tₘₐₓ) is 1.3 hours (Fig. 8), the area under the blood level-time curve (AUC) is 64.5 ng.hr/ml (Example 5), the disintegration time is between 4 and 6 minutes (Example 5), and the time necessary for 100% dissolution is 150 minutes (Fig. 7). The term "absorption" or "in vivo absorption" as used herein means the inherent in vivo absorption of the active agent, i.e. in vivo absorption characterized by traditional non-sustained release preparations, and the absorption can be represented by the time course of blood level of the active agent. Herein, the traditional medicinal preparation refers to a non-sustained release preparation.

The SD tablet of this invention is also characterized by being less dependent on pH during the dissolution of a medicament in stomach (Fig. 9).

The above-described characteristics of the SD tablet according to the present invention are unique and differ from those of the solid dispersion material (Comparative Example 2) which was obtained by simultaneously mixing the three essential components, i.e. a substantially water-insoluble 1,4-dihydropyridine derivative represented by the general formula [I], hydroxypropyl cellulose and hydroxypropyl methyl cellulose phthalate, or from those of the solid dispersion materials (a) or (b) alone (Example 5). Particularly, the fact that the SD tablet of the present invention does not alter the inherent absorption of the active agent indicates that the side effects caused in association with the maintenance of a blood level of the active agent can be avoided.

The 1,4-dihydropyridine derivative-containing SD tablet of the present invention can be prepared by the method which comprises the following steps of:
(i) dissolving a 1,4-dihydropyridine derivative as a pharmaceutical active agent represented by the general formula [I] as defined in claim 1 or a pharmaceutically acceptable acid addition salt thereof and hydroxypropyl cellulose in an organic solvent, if desired adding and mixing one or more auxiliaries chosen from excipients and disintegrating agents, and then removing the organic solvent from the mixture, for example by evaporation, to produce a solid dispersion material (a);
(ii) dissolving said 1,4-dihydropyridine derivative or its pharmaceutically acceptable acid addition salt and hydroxypropyl methyl cellulose phthalate in the organic solvent, if desired adding and mixing one or more auxiliaries chosen from excipients and disintegrating agents, and then removing the organic solvent from the mixture, for example by evaporation, to produce a solid dispersion material (b);
(iii) mixing together said solid dispersion material (a), said solid dispersion material (b), and one or more optional lubricants; and
(iv) compressing the resulting mixture to form into tablets.

When the solid dispersion material(s) contains an excipient, magnesium methasilicate aluminate is desirable as excipient. In this case, the solid dispersion materials (a) and (b) may contain magnesium methasilicate aluminate such that the weight ratio of a 1,4- dihydropyridine derivative or its acid addition salt to magnesium methasilicate aluminate is 1:11 or more, preferably 1:11 to 1:12 in the (a), and 1:5.5 or more, preferably 1:5.5 to 1:12 in the (b).

The organic solvent includes, for example, lower alcohols such as methanol and ethanol, halogenated hydrocarbons such as dichloromethane and chloroform, or mixtures thereof.

Molding into tablets can be effected by the conventional procedures.

Thus, the above-described process is also included within the scope of the invention.

When the SD tablet of the present invention is administered to animals, especially human, the dosage is generally selected depending on patient's age, symptom and body weight, known permisible doses determined for known active agents, and so on.

As seen from the above description, the medicinal preparation of the present invention should be in the form of tablet, thereby resulting in different properties from the mixed granules of solid dispersion materials (a)/(b) = 3/7 (Comparative Example 3).

### Advantages of the Invention

The 1,4-dihydropyridine derivative-containing SD tablet according to the present invention advantageously has less pH-dependency during dissolution of the active agent in stomach, and has as high in vivo absorption as the HPC.SD tablets while retaining the inherent absorption of the active agent. Accordingly, the SD tablet of this invention improves the bioavailability of the 1,4-dihydropyridine derivative which is substantially insoluble in water, as well as the avoidance of side effects.

The present invention is illustrated by the following comparative examples and working examples where FRC8653 (Japanese Patent Application Laying open (KOKAI) No. 60-233058) is taken as an example of the 1,4-dihydropyridine derivative represented by the general formula [I], FRC8653 being an antihypertensive agent with Ar = m-nitrophenyl; R¹ = methyl; R² = cinnamyl; and both R³ and R⁴ = methyl in the formula [I]. In this case, it should be understood that other derivatives can also be applied to the production of medicinal preparations of the present invention.

### Comparative Example 1

### Wet granule-compression tablets

Relative to one tablet (135 mg), 5.0 mg of FRC8653 (volume average diameter, 50 µm or less) was mixed with the following materials in water, granulated and then dried to product wet granules: an adequate amount of lactose; 12.5 mg of corn starch and 18.7 mg of crystalline cellulose as excipients; 12.5 mg of hydroxypropyl cellulose with a low degree of substitution as a disintegrating agent; and 3.7 mg of hydroxypropyl cellulose (Nippon Soda, Japan; referred to as HPC hereinafter) as a binder. To the wet granules were further added 1.3 mg of talc and 1.2 mg of magnesium stearate as lubricants followed by molding into tablets. The resulting tablets were film-coated with the solution of 5.0 mg of hydroxypropyl methyl cellulose 2910 (6cs) (Shin-Etsu Chemical Co., Japan) and 1.25 mg of ethyl cellulose (Hercules) as coating agents; 0.625 mg of Macrogol 600 (Nippon Oil and Fats Co., Japan) as a placiticizer; and 2.5 mg of titanium oxide as an opaquing agent in the mixed solvent of methanol/dichloromethane, to produce wet granule-compression tablets.

The wet granule-compression tablets prepared were tested for disintegration and dissolution. As the results, the disintegration time measured by the disintegration test of Japanese Official Standards (Nikkyoku) was between 3.0 and 5.5 minutes, and the values of % dissolution measured by the dissolution test (2nd method) of Japanese Official Standards were about 20% at 10 min, about 35% at 30 min, about 50% at 60 min, about 60% at 90 min, and about 70% at 150 min. In this case, a test solution of 0.5% (w/v) polysorbate 80 in a 1st test solution for disintegration test of Japanese Official Standards was used in order to achieve at least 5-fold the saturated solubility of FRC 8653 in water at 37°C, the total volume of the test solution was 500ml, and the paddle rotation was 100 rounds per minutes.

Moreover, after the wet granule-compression tablets were orally administered to dogs in the amount of 10 mg (2 tablets) of FRC8653 per body, the blood level of FRC8653 was determined using high performance liquid chromatography (chromatography conditions: ODS column; acetonitrile/water system). As a result, the maximum blood level at 1.2 hours after oral administration was 5.3 ng/ml (Fig. 1). The pharmacokinetic parameters calculated from the blood level-time curve were: time required to reach the maximum blood level (tₘₐₓ), 1.2 hours; half life time (t_{1/2}), 1.2 hours; and area under the blood level-time curve (AUC), 14.1 ng.hr/ml. FRC8653 was dissolved in the mixed solvent (1/1) of ethanol and Nikkol (HCO-60) (Nikko Chemicals, Japan), and the resulting solution was diluted to 1:50 with physiological saline and intravenously injected into the animal in order to determine AUC values. When the bioavailability was calculated based on the AUC determined, it was only 5.3%.

### Comparative Example 2

Relative to one tablet (135 mg), the solution which was prepared by simultaneously dissolving 5.0 mg of FRC8653 (volume average diameter, 50µm or less), 6.0 mg of HPC as binder (carrier), and 14.0 mg of hydroxypropyl methyl cellulose phthalate (Shin-Etsu Chemical; referred to as HP55 hereinafter) in 110.0 mg of the mixed solvent of methanol/dichloromethane, was mixed with an adequate amount of lactose, 15.7 mg of crystalline cellulose, 3.5 mg of Macrogol 400 (Nippon Oil and Fats Co.) and 55.0 mg of magnesium methasilicate aluminate as excipients, and 12.0 mg of sodium croscarmellose (Asahi Chemical Industry, Japan) as disintegrating agent, with stirring. After the removal of the solvent by evaporation, 124.0 mg of the HPC/HP55 (3/7) solid dispersion (SD) granules was produced. Then, to the granules were further added 0.5 mg of talc and 0.5 mg of magnesium stearate as lubricants, followed by molding into tablets. The resulting tablets were film-coated under the same conditions as in the preceding Comparative Example to produce HPC/HP55 (3/7) SD tablets. As shown in Fig. 2., the results of dissolution test of the tablets prepared indicate that the preparation from the simple mixing of the three essential components, i.e. FRC8653, HPC and HP55, has significantly poor dissolution compared with the medicinal preparation of the present invention (HPC/HP55 mixed (3/7) SD tablets prepared in Example 5). Accordingly, it is assumed that the preparation of this comparative example has poorer in vivo absorption.

### Example 1

### Determination of an amount of HPC relative to FRC8653 in the case of the production of mixed solid dispersion tablets (HPC/HP55 mixed SD tablets) with HPC and HP55 as carriers

HPC and FRC8653, in the propotion of 1, 2, 3, 4 or 5 parts by weight of HPC based on one part by weight of FRC8653, were added to the mixed solvent of methanol/dichloromethane and dissolved, followed by removal of the solvent and drying. The samples obtained were subjected to thermal analysis (DSC). As shown in Fig. 3, the amount of HPC required to convert FRC8653 into its amorphous form was 4 parts by weight or more per one part by weight of FRC8653.

### Example 2

### Production of HPC.SD granules

Relative to one tablet (135 mg), the solution of 5.0 mg of FRC8653 (volume average diameter, 50 µm or less) and 20.0 mg of HPC as binder (carrier) in 75 mg of the mixed solvent of methanol/dichloromethane was added to an adequate amount of lactose, 15.7 mg of crystalline cellulose, 3.5 mg of Macrogol 400 (Nippon Oil and Fats Co.) and 55.0 mg of magnesium methasilicate aluminate as excipients, and 12.0 mg of sodium croscarmellose (Asahi Chemical Industry) as a disintegrating agent, and then mixed with stirring. After the removal of the solvent by evaporation, 124.0 mg of HPC.SD granules were produced.

For comparison, HPC.SD tablets were prepared in the following manner and their performance or properties were then examined:

To the HPC.SD granules obtained above were added 0.5 mg of talc and 0.5 mg of magnesium stearate as lubricants, followed by molding into tablets. The resulting tablets were film-coated under the same conditions as in the above Comparative Examples to produce HPC.SD tablets.

In the same way as in Comparative Example 1, the disintegration time, % dissolution, blood level and pharmacokinetic parameters of the HPC.SD tablets prepared were determined. As the results, the disintegration time was between 4.0 and 5.5 minutes which value indicates the good disintegration of the HPC.SD tablets, and the time necessary for 100% dissolution was as short as 30 minutes, thus suggesting a very high dissolution rate (Fig. 4). The time course of blood levels of FRC8653 when orally administered to dogs is shown in Fig. 5. The pharmacokinetic parameters calculated from the figure were: tₘₐₓ, 30 minutes or less; t_{1/2}, 1.9 hours; and AUC, 64.4 ng.hr/ml. Such a rapid dissolution clearly deviates from the inherent in vivo absorption of FRC8653 as shown in Fig. 1.

### Example 3

### Determination of an amount of HP55 relative to FRC8653 in the case of the production of HPC/HP55 mixed SD tablets with HPC and HP55 as carriers

Both HP55 (Shin-Etsu Chemical Co.) and FRC8653, in the proportion of 1, 2, 3, 4 or 5 parts by weight of HP55 based on one part by weight of FRC8653, were added to the mixed solvent of methanol/dichloromethane and dissolved, followed by removal of the solvent and drying in order to subject to thermal analysis (DSC). The results are shown in Fig. 6. The amount of HP55 necessary to convert the FRC8653 into its amorphous form was 2 parts by weight or more per one part by weight of FRC8653.

### Example 4

### Production of HP55.SD granules

The solution of 5.0 mg of FRC8653 (volume average diameter, 50µm or less) and 20.0 mg of HP55 as binder in 125 mg of the mixed solvent of methanol/dichloromethane was added to an adequate amount of lactose, 15.7 mg of crystalline cellulose, 3.5 mg of Macrogol 400 and 55.0 mg of magnesium methasilicate aluminate as excipients, and 12.0 mg of sodium croscarmellose as disintegrating agent, and then mixed with stirring. After the removal of the solvent by evaporation, 124.0 mg of HP55.SD granules were produced.

### Example 5

### Production of HPC/HP55 mixed SD tablets

The HPC.SD granules and HP55.SD granules produced in accordance with the procedures set forth in Examples 2 and 4 respectively were mixed in the ratio of 10/0, 7/3, 3/7 or 0/10 (weight/weight). Then, in the same way as in the production of the HP.SD tablets (Example 2), the aforesaid lubricants were added to each of the mixtures which was then molded into tablets and film-coated to produce HPC/HP55 mixed SD tablets.

For the resulting HPC/HP55 mixed (10/0) SD tablets, HPC/HP55 mixed (7/3) SD tablets (the present invention), HPC/HP55 mixed (3/7) SD tablets (the present invention) and HPC/HP55 mixed (0/10) SD tablets, disintegration and dissolution were determined. All the disintegration times determined of these SD tablets were between 4.0 and 6.0 minutes which values indicate their good disintegration. The % dissolution of FRC8653, as shown in Fig. 7, was 100% at 30 minutes in the case of the HPC/HP55 mixed (10/0) SD tablets; 100% at 2.5 hours in the case of the HPC/HP55 mixed (3/7) SD tablets; 86% at 2.5 hours in the case of the HPC/HP55 mixed (0/10) SD tablets; and 100% at 1.5 hours in the case of the HPC/HP55 mixed (7/3) SD tablets, provided that the testing time of dissolution was not more than 2.5 hours on the assumption that each of the preparations would stay for 2.5 hours in stomach after oral administeration.

Furthermore, the HPC/HP55 mixed (3/7) SD tablets which have been believed to be suitable for an antihypertensive FRC8653-containing medicinal preparation based on the results of % dissolution described above were orally administered to dogs at the dose of 10 mg of FRC8653 per body, and the blood levels of FRC8653 were determined by high performance liquid chromatography under the same conditions as in Comparative Example 1 (Fig. 8). The pharmacokinetic parameters were calculated from the blood level-time curve in the figure. As the results, tₘₐₓ was 1.3 hours, t_{1/2} was 1.8 hours, and AUC was 64.5 ng.hr/ml. The tₘₐₓ was nearly equal to that of the wet granule-compression tablets (Comparative Example 1), and the AUC to that of the HPC.SD tablets (Example 2).

### Example 6

### Dissolution profiles of HPC/HP55 mixed (3/7) SD tablets under acidic or neutral conditions

For the HPC/HP55 mixed (3/7) so tablets and HPC/HP55 mixed (0/10) SD tablets (each containing 5mg of FRC8653), the dissolution behaviors of FRC8653 were measured in each 500ml of test solutions of pH 1.2 (0.5% (w/v) polysorbate 80 / the 1st test solution of Japanese Official Standards) and of pH 7.2 (0.5% (w/v) polysorbate 80 / phosphate buffer) at paddle rotation of 100 R.P.M. (Fig. 9).

From Fig. 9, it was found that in the case of the HPC/HP55 mixed (3/7) SD tablets the values of % dissolution of FRC8653 after 90 minutes were 85% at pH 1.2 and 95% at pH 7.2, thus being less pH-dependent, while in the case of the HPC/HP55 mixed (0/10) SD tablets its values were 65% at pH 1.2 and 100% at pH 7.2.

### Comparative Example 3

For 124.0 mg of the mixed granules obtained by mixing the HPC.SD granules and HP55.SD granules which have been prepared by the methods set forth in Examples 2 and 4, respectively, in the ratio of 3/7 (weight/weight), and for the HPC/HP55 mixed (3/7) SD tablets prepared in Example 5, the values of % dissolution were determined in the same way as in Comparative Example 1. As shown in Fig. 10, the % dissolution of the mixed granules was about 75% within 150 minutes, and this value was inferior to 100% dissolution of the HPC/HP55 mixed (3/7) SD tablets. Accordingly, the in vivo absorption of said mixed granules is also thought to be inferior to that of the HPC/HP55 mixed (3/7) SD tablets according to the present invention.

## Claims (Claims for the following Contracting State(s): AT, BE, DK, SE)

1. A pharmaceutical solid dispersion (SD) tablet comprising a solid dispersion material (a) which comprises a substantially water-insoluble 1,4-dihydropyridine derivative as in amorphous form a pharmaceutical active agent represented by the general formula [I]: 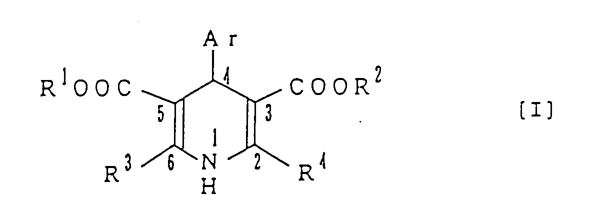 wherein Ar is a substituted aryl or benzofurazanyl group, R¹ is a substituted or unsubstituted alkyl group, R² is a substituted or unsubstituted alkyl or alkenyl group or a substituted piperidyl group, R³ and R⁴ each independently represent a substituted or unsubstituted alkyl group, and the substituent R¹OCO- at the 5-position may be replaced by a dialkylphosphonoate or alkylene dioxyphosphoryl group, or a pharmaceutically acceptable acid addition salt thereof and hydroxypropyl cellulose; and a solid dispersion material (b) which comprises said 1,4-dihydropyridine derivative in amorphous form or its pharmaceutically acceptable acid addition salt and hydroxypropyl methyl cellulose phthalate.

2. A pharmaceutical SD tablet according to claim 1, wherein the weight ratio of the 1,4-dihydropyridine derivative or its pharmaceutically acceptable acid addition salt to the hydroxypropyl cellulose is from 1:4 to 1:5, and the weight ratio of the 1,4-dihydropyridine derivative or its acid addition salt to the hydroxypropyl methyl cellulose phthalate is from 1:2 to 1:5.

3. A pharmeceutical SD tablet according to claim 1, wherein the mixing ratio of the solid dispersion material (a) to the solid dispersion material (b) is from 2.5:7.5 to 3.5:6.5 by weight.

4. A pharmaceutical SD tablet according to claim 1, wherein the solid dispersion materials (a) or (b) further comprises an apropriate amount of one or more auxiliaries chosen from excipients, disintegrating agents and lubricants.

5. A pharmaceutical SD tablet according to claim 4, wherein the auxiliary is magnesium methasilicate aluminate.

6. A pharmaceutical SD tablet according to claim 5, wherein the magnesium methasilicate aluminate is incorporated into the solid dispersion materials (a) or (b) in the weight ratio of the 1,4-dihydropyridine derivative or its pharmaceutically acceptable acid addition salt to the magnesium methasilicate aluminate of from 1:11 to 1:12 or of from 1:5.5 to 1:12, respectively.

7. A process for the preparation of a pharmaceutical SD tablet according to any one of claims 1 to 6, which comprises the following steps of:
(i) dissolving a 1,4-dihydropyridine derivative as a pharmaceutical active agent represented by the general formula [I] as defined in claim 1 or a pharmaceutically acceptable acid addition salt thereof and hydroxypropyl cellulose in an organic solvent, if desired adding and mixing one or more auxiliaries chosen from excipients and disintegrating agents, and then removing the organic solvent from the mixture to produce a solid dispersion material (a);
(ii) dissolving said 1,4-dihydropyridine derivative or its pharmaceutically acceptable acid addition salt and hydroxypropyl methyl cellulose phthalate in the organic solvent, if desired adding and mixing one or more auxiliaries chosen from excipients and disintegrating agents, and then removing the organic solvent from the mixture to produce a solid dispersion material (b);
(iii) mixing together said solid dispersion material (a), said solid dispersion material (b), and one or more optional lubricants; and
(iv) compression-molding the resulting mixture into tablets.

8. A process according to claim 7, which further comprises a step of film-coating the tablet with a coating agent.

9. A process according to claims 7 or 8, wherein the organic solvent is a lower alcohol, halogenated hydrocarbon, or mixture thereof.

## Claims (Claims for the following Contracting State(s): ES)

1. A process for preparing a pharmaceutical solid dispersion (SD) tablet comprising a solid dispersion material (a) which comprises a substantially water-insoluble 1,4-dihydropyridine derivative in amorphous form as a pharmaceutical active agent represented by the general formula [I]: 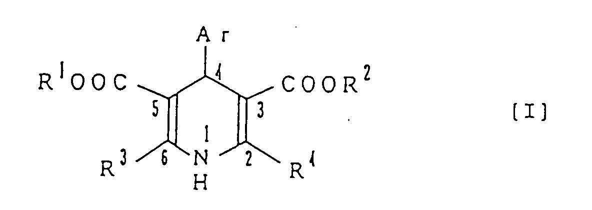 wherein Ar is a substituted aryl or benzofurazanyl group, R¹ is a substituted or unsubstituted alkyl group, R² is a substituted or unsubstituted alkyl or alkenyl group or a substituted piperidyl group, R³ and R⁴ each independently represent a substituted or unsubstituted alkyl group, and the substituent R¹OCO- at the 5-position may be replaced by a dialkylphosphonoate or alkylene dioxyphosphoryl group, or a pharmaceutically acceptable acid addition salt thereof and hydroxypropyl cellulose; and a solid dispersion material (b) which comprises said 1,4-dihydropyridine derivative in amorphous form or its pharmaceutically acceptable acid addition salt and hydroxypropyl methyl cellulose phthalate, the process comprising the steps of:
(i) dissolving a 1,4-dihydropyridine derivative as a pharmaceutical active agent represented by the above general formula [I] or a pharmaceutically acceptable acid addition salt thereof and hydroxypropyl cellulose in an organic solvent, if desired adding and mixing one or more auxiliaries chosen from excipients and disintegrating agents, and then removing the organic solvent from the mixture to produce a solid dispersion material (a);
(ii) dissolving said 1,4-dihydropyridine derivative or its pharmaceutically acceptable acid addition salt and hydroxypropyl methyl cellulose phthalate in the organic solvent, if desired adding and mixing one or more auxiliaries chosen from excipients and disintegrating agents, and then removing the organic solvent from the mixture to produce a solid dispersion material (b);
(iii) mixing together said solid dispersion material (a), said solid dispersion material (b), and one or more optional lubricants; and
(iv) compression-molding the resulting mixture into tablets.

2. A process according to claim 1, which further comprises the step of film-coating the tablet with a coating agent.

3. A process according to claim 1 or claim 2 wherein the organic solvent is a lower alcohol, halogenated hydrocarbon, or mixture thereof.

4. The use of a mixture of a solid dispersion material (a) which comprises a substantially water-insoluble 1,4-dihydropyridine derivative in amorphous form as a pharmaceutical active agent represented by the general formula [I]: 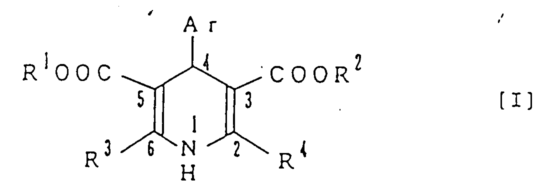 wherein Ar is a substituted aryl or benzofurazanyl group, R¹ is a substituted or unsubstituted alkyl group, R² is a substituted or unsubstituted alkyl or alkenyl group or a substituted piperidyl group, R³ and R⁴ each independently represent a substituted or unsubstituted alkyl group, and the substituent R¹OCO- at the 5-position may be replaced by a dialkylphosphonoate or alkylene dioxyphosphoryl group, or a pharmaceutically acceptable acid addition salt thereof and hydroxypropyl cellulose; and a solid dispersion material (b) which comprises said 1,4-dihydropyridine derivative in amorphous form or its pharmaceutically acceptable acid addition salt and hydroxypropyl methyl cellulose phthalate; for the manufacture of a vasodilating SD tablet.

5. The use according to claim 4, wherein the weight ratio of the 1,4-dihydropyridine derivative or its pharmaceutically acceptable acid addition salt to the hydroxypropyl cellulose is from 1:4 to 1:5, and the-weight ratio of the 1,4-dihydropyridine derivative or its acid addition salt to the hydroxypropyl methyl cellulose phthalate is from 1:2 to 1:5.

6. The use according to claim 4, wherein the mixing ratio of the solid dispersion material (a) to the solid dispersion material (b) is from 2.5:7.5 to 3.5:6.5 by weight.

7. The use according to claim 4, wherein the solid dispersion materials (a) or (b) further comprises an apropriate amount of one or more auxiliaries chosen from excipients, disintegrating agents and lubricants.

8. The use according to claim 7, wherein the auxiliary is magnesium methasilicate aluminate.

9. The use according to claim 8, wherein the magnesium methasilicate aluminate is incorporated into the solid dispersion materials (a) or (b) in the weight ratio of the 1,4-dihydropyridine derivative or its pharmaceutically acceptable acid addition salt to the magnesium methasilicate aluminate of from 1:11 to 1:12 or of from 1:5.5 to 1:12, respectively.

## Patentansprüche (Patentansprüche für folgende(n) Vertragsstaat(en): AT, BE, DK, SE)

1. Pharmazeutische Feststoffdispersions-(FD)-Tablette, umfassend ein Feststoffdispersionsmaterial (a), welches ein im wesentlichen wasserunlösliches 1,4-Dihydropyridin-Derivat in amorpher Form als pharmazeutisch wirksames Mittel, dargestellt durch die allgemeine Formel [I]: 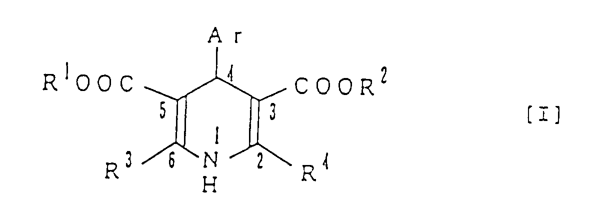 wobei Ar eine substituierte Aryl- oder Benzofurazanyl-Gruppe darstellt, R¹ eine substituierte oder unsubstituierte Alkyl-Gruppe darstellt, R² eine substituierte oder unsubstituierte Alkyl- oder Alkenyl-Gruppe oder eine substituierte Piperidyl-Gruppe darstellt, R³ und R⁴ unabhängig voneinander eine substituierte oder unsubstituierte Alkyl-Gruppe darstellen, und der Substituent R¹OCO- an der 5-Position durch eine Dialkylphosphonat- oder Alkylendioxyphosphoryl-Gruppe ersetzt werden kann, oder ein pharmazeutisch annehmbares Säureadditionssalz hiervon und Hydroxypropylcellulose umfaßt, und ein Feststoffdispersionsmaterial (b), welches besagtes 1,4-Dihydropyridin-Derivat in amorpher Form oder sein pharmazeutisch annehmbares Säureadditionssalz und Hydroxypropylmethylcellulosephthalat umfaßt.

2. Pharmazeutische FD-Tablette gemäß Anspruch 1, in der das Gewichtsverhältnis des 1,4-Dihydropyridin-Derivats oder seines pharmazeutisch annehmbaren Säureadditionssalzes zu der Hydroxypropylcellulose 1:4 bis 1:5 beträgt und das Gewichtsverhältnis des 1,4-Dihydropyridin-Derivats oder seines Säureadditionssalzes zu Hydroxypropylmethylcellulosephthalat 1:2 bis 1:5 beträgt.

3. Pharmazeutische FD-Tablette gemäß Anspruch 1, in der das Mischverhältnis des Feststoffdispersionsmaterials (a) zu dem Feststoffdispersionsmaterial (b) 2,5:7,5 bis 3,5:6,5, bezogen auf das Gewicht, beträgt.

4. Pharmazeutische FD-Tablette gemäß Anspruch 1, in der die Feststoffdispersionsmaterialien (a) oder (b) weiterhin eine angemessene Menge eines oder mehrerer Hilfsstoffe ausgewählt aus Arzneimittelträgern, Aufschlußmitteln und Gleitmitteln umfassen.

5. Pharmazeutische FD-Tablette gemäß Anspruch 4, in der der Hilfsstoff Magnesiummetasilicataluminat ist.

6. Pharmazeutische FD-Tablette gemäß Anspruch 5, in der das Magnesiummetasilicataluminat in den Feststoffdispersionsmaterialien (a) oder (b) in einem Gewichtsverhältnis des 1,4-Dihydropyridin-Derivats oder seines pharmazeutisch annehmbaren Säureadditionssalzes zu dem Magnesiummetasilicataluminat jeweils von 1:11 bis 1:12 oder von 1:5,5 bis 1:12 enthalten ist.

7. Verfahren zur Herstellung einer pharmazeutisch FD-Tablette gemäß einem der Ansprüche 1 bis 6, welches die folgenden Schritt umfaßt:
(i) Auflösen eines 1,4-Dihydropyridin-Derivats als pharmazeutisch wirksames Mittel, dargestellt durch die allgemeine Formel [I], wie in Anspruch 1 definiert, oder eines pharmazeutisch annehmbaren Säureadditionssalzes hiervon und von Hydroxypropylcellulose in einem organischen Lösungsmittel, falls erwünscht Zufügen von und Mischen mit einem oder mehreren Hilfsstoffen ausgewählt aus Arzneimittelträgern und Aufschlußmitteln und anschließend Entfernen des organischen Lösungsmittels aus der Mischung, beispielsweise durch Verdampfung, um ein Feststoffdispersionsmaterial (a) herzustellen;
(ii) Auflösen des 1,4-Dihydropyridin-Derivats oder seines pharmazeutisch annehmbaren Säureadditionssalzes und von Hydroxypropylmethylcellulosephthalat in dem organischen Lösungsmittel, falls erwünscht Zufügen von und Mischen mit einem oder mehreren Hilfsstoffen, ausgewählt aus Arzneimittelträgern und Aufschlußmitteln, und anschließend Entfernen des organischen Lösungsmittels aus der Mischung, beispielsweise durch Verdampfung, um ein Feststoffdispersionsmaterial (b) herzustellen;
(iii) Zusammenmischen des Feststoffdispersionsmaterials (a), des Feststoffdispersionsmaterials (b) und eines oder mehrerer optionaler Gleitmittel; und
(iv) Komprimieren der resultierenden Mischung, um Tabletten zu bilden.

8. Verfahren gemäß Anspruch 7, welches weiterhin einen Schritt des Filmbeschichtens der Tablette mit einem Beschichtungsmittel umfaßt.

9. Verfahren gemäß Anspruch 7 oder 8, in dem das organische Lösungsmittel ein Niederalkohol, ein halogenierte Kohlenwasserstoff oder eine Mischung hiervon ist.

## Patentansprüche (Patentansprüche für folgende(n) Vertragsstaat(en): ES)

1. Verfahren zur Herstellung einer pharmazeutischen Feststoffdispersions-(FD)-Tablette, umfassend ein Feststoffdispersionsmaterial (a), welches ein im wesentlichen wasserunlösliches 1,4-Dihydropyridin-Derivat in amorpher Form als pharmazeutisch wirksames Mittel, dargestellt durch die allgemeine Formel [I]: 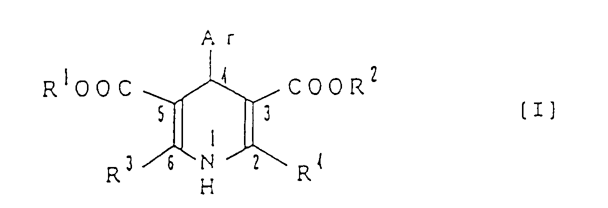 wobei Ar eine substituierte Aryl- oder Benzofurazanyl-Gruppe darstellt, R¹ eine substituierte oder unsubstituierte Alkyl-Gruppe darstellt, R² eine substituierte oder unsubstituierte Alkyl- oder Alkenyl-Gruppe oder eine substituierte Piperidyl-Gruppe darstellt, R³ und R⁴ unabhängig voneinander eine substituierte oder unsubstituierte Alkyl-Gruppe darstellen, und der Substituent R¹OCO- an der 5-Position durch eine Dialkylphosphonat- oder Alkylendioxyphosphoryl-Gruppe ersetzt werden kann, oder ein pharmazeutisch annehmbares Säureadditionssalz hiervor und Hydroxypropylcellulose umfaßt, und ein Feststoffdispersionsmaterial (b), welches besagtes 1,4-Dihydropyridin-Derivat in amorpher Form oder sein pharmazeutisch annehmbares Säureadditionssalz und Hydroxypropylmethylcellulosephthalat umfaßt, wobei das Verfahren die Schritt umfaßt:
(i) Auflösen eines 1,4-Dihydropyridin-Derivats als pharmazeutisch wirksames Mittel, dargestellt durch die allgemeine Formel [I], wie in Anspruch 1 definiert, oder eines pharmazeutisch annehmbaren Säureadditionssalzes hiervon und von Hydroxypropylcellulose in einem organischen Lösungsmittel, falls erwünscht Zufügen von und Mischen mit einem oder mehreren Hilfsstoffen ausgewählt aus Arzneimittelträgern und Aufschlußmitteln und anschließend Entfernen des organischen Lösungsmittels aus der Mischung, beispielsweise durch Verdampfung, um ein Feststoffdispersionsmaterial (a) herzustellen;
(ii) Auflösen des 1,4-Dihydropyridin-Derivats oder seines pharmazeutisch annehmbaren Säureadditionssalzes und von Hydroxypropylmethylcellulosephthalat in dem organischen Lösungsmittel, falls erwünscht Zufügen von und Mischen mit einem oder mehreren Hilfsstoffen, ausgewählt aus Arzneimittelträgern und Aufschlußmitteln, und anschließend Entfernen des organischen Lösungsmittels aus der Mischung, beispielsweise durch Verdampfung, um ein Feststoffdispersionsmaterial (b) herzustellen;
(iii) Zusammenmischen des Feststoffdispersionsmaterials (a), des Feststoffdispersionsmaterials (b) und eines oder mehrerer optionaler Gleitmittel; und
(iv) Komprimieren der resultierenden Mischung, um Tabletten zu bilden.

2. Verfahren gemäß Anspruch 1, welches weiterhin einen Schritt des Filmbeschichtens der Tablette mit einem Beschichtungsmittel umfaßt.

3. Verfahren gemäß Anspruch 1 oder 2, indem das organische Lösungsmittel ein Niederalkohol, ein halogenierter Kohlenwasserstoff oder eine Mischung hiervon ist.

4. Verwendung einer Mischung eines Feststoffdispersionsmaterials (a), welches ein im wesentlichen wasserunlösliches 1,4-Dihydropyridin-Derivat in amorpher Form als pharmazeutisch wirksames Mittel, dargestellt durch die allgemeine Formel [I]: 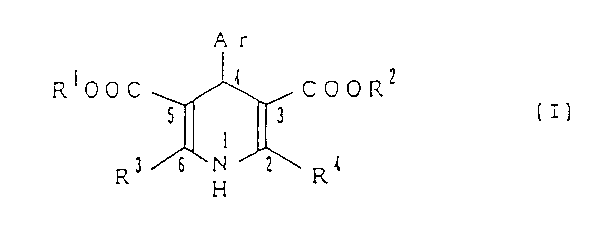 wobei Ar eine substituierte Aryl- oder Benzofurazanyl-Gruppe darstellt, R¹ eine substituierte oder unsubstituierte Alkyl-Gruppe darstellt, R² eine substituierte oder unsubstituierte Alkyl- oder Alkenyl-Gruppe oder eine substituierte Piperidyl-Gruppe darstellt, R³ und R⁴ unabhängig voneinander eine substituierte oder unsubstituierte Alkyl-Gruppe darstellen, und der Substituent R¹OCO- an der 5-Position durch eine Dialkylphosphonat- oder Alkylendioxyphosphoryl-Gruppe ersetzt werden kann, oder ein pharmazeutisch annehmbares Säureadditionssalz hiervon und Hydroxypropylcellulose umfaßt, und ein Feststoffdispersionsmaterial (b), welches besagtes 1,4-Dihydropyridin-Derivat in amorpher Form oder sein pharmazeutisch annehmbares Säureadditionssalz und Hydroxypropylmethylcellulosephthalat umfaßt; zur Herstellung einer gefäßerweiternden FD-Tablette.

5. Verwendung gemäß Anspruch 4, wobei das Gewichtsverhältnis des 1,4-Dihydropyridin-Derivats oder seines pharmazeutisch annehmbaren Säureadditionssalzes zu der Hydroxypropylcellulose 1:4 bis 1:5 beträgt und das Gewichtsverhältnis des 1,4-Dihydropyridin-Derivats oder seines Säureadditionssalzes zu Hydroxypropylmethylcellulosephthalat 1:2 bis 1:5 beträgt.

6. Verwendung gemäß Anspruch 4, wobei das Mischungsverhältnis des Feststoffdispersionsmaterials (a) zu dem Feststoffdispersionsmaterial (b) 2,5:7,5 bis 3,5:6,5, bezogen auf das Gewicht, beträgt.

7. Verwendung gemäß Anspruch 4, wobei die Feststoffdispersionsmaterialien (a) oder (b) weiterhin eine angemessene Menge eines oder mehrerer Hilfsstoffe ausgewählt aus Arzneimittelträgern, Aufschlußmitteln und Gleitmitteln umfassen.

8. Verwendung gemäß Anspruch 7, wobei der Hilfsstoff Magnesiummetasilicataluminat ist.

9. Verwendung gemäß 8, wobei das Magnesiummetasilicataluminat in den Feststoffdispersionsmaterialien (a) oder (b) in einem Gewichtsverhältnis des 1,4-Dihydropyridin-Derivats oder seines pharmazeutisch annehmbaren Säureadditionssalzes zu dem Magnesiummetasilicataluminat jeweils von 1:11 bis 1:12 oder von 1:5,5 bis 1:12 enthalten ist.

## Revendications (Revendications pour l'(les) Etat(s) contractant(s) suivant(s): AT, BE, DK, SE)

1. Comprimé à dispersion solide (DS) pharmaceutique, comprenant une matière en dispersion solide (a) qui comprend un dérivé de la 1,4-dihydropyridine, sous forme amorphe, sensiblement insoluble dans l'eau, à titre d'agent pharmaceutique actif, représenté par la formule générale [I] 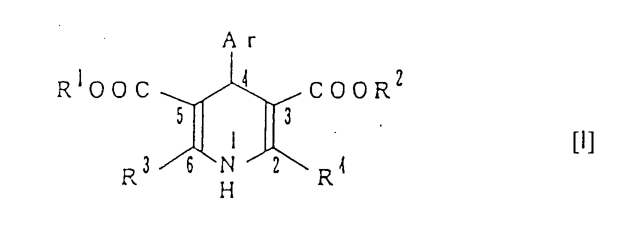 dans laquelle Ar représente un radical benzofurazannyle ou aryle substitué, R¹ représente un radical alkyle substitué ou non substitué, R² représente un radical alcényle ou un radical alkyle substitué ou non substitué, ou un groupe pipéridyle substitué, R³ et R⁴ représentent chacun indépendamment un radical alkyle substitué ou non substitué et le substituant R¹OCO- en position 5 peut être remplacé par un groupe dialkylphosphonate ou alkylènedioxyphosphoryle, ou un sel d'addition d'acide pharmaceutiquement acceptable d'un tel composé et de l'hydropropylcellulose et une matière en dispersion solide (b) qui comprend ledit dérivé de la 1,4-dihydropyridine, sous forme amorphe, ou son sel d'addition d'acide pharmaceutiquement acceptable et de l'hydroxypropylméthylcellulosephtalate.

2. Comprimé DS pharmaceutique suivant la revendication 1, caractérisé en ce que le rapport pondéral du dérivé de la 1,4-dihydropyridine ou de son sel d'addition d'acide pharmaceutiquement acceptable à l'hydroxypropylcellulose varie de 1:4 à 1:5 et le rapport pondéral du dérivé de la 1,4-dihydropyridine ou de son sel d'addition d'acide à l'hydroxypropylméthylcellulosephtalate varie de 1:2 à 1:5.

3. Comprimé DS pharmaceutique suivant la revendication 1, caractérisé en ce que le rapport de mélange de la matière en dispersion solide (a) vis-à-vis de la matière en dispersion solide (b) varie de 2,5:7,5 à 3,5:6,5 en poids.

4. Comprimé DS pharmaceutique suivant la revendication 1, caractérisé en ce que les matières en dispersion solide (a) ou (b) comprennent en outre une quantité appropriée d'un ou plusieurs auxiliaires choisis parmi les excipients, des agents de désagrégation et des lubrifiants.

5. Comprimé suivant la revendication 4, caractérisé en ce que l'auxiliaire est l'aluminate-méthasilicate de magnésium.

6. Comprimé suivant la revendication 5, caractérisé en ce que l'on incorpore l'aluminate-méthasilicate de magnésium aux matières en dispersion solide (a) ou (b) dans le rapport pondéral du dérivé de la 1,4-dihydropyridine ou de son sel d'addition d'acide pharmaceutiquement acceptable à l'aluminate-méthasilicate de magnésium de 1:11 à 1:12 ou de 1:5,5 à 1:12, respectivement.

7. Procédé pour la préparation d'un comprimé DS pharmaceutique suivant l'une quelconque des revendications 1 à 6, comprenant les étapes consistant à :
(i) dissoudre le dérivé de la 1,4-dihydropyridine à titre d'agent pharmaceutique actif et représenté par la formule générale [I], telle que définie dans la revendication 1, ou un sel d'addition d'acide pharmaceutiquement acceptable de celui-ci et de l'hydroxypropylcellulose dans un solvant organique, si cela se révèle souhaitable, ajouter et mélanger un ou plusieurs auxiliaires choisis parmi des excipients et des agents de désagrégation et ensuite chasser le solvant organique du mélange, par exemple, par évaporation, pour produire une matière en dispersion solide (a),
(ii) dissoudre ledit dérivé de la 1,4-dihydropyridine ou son sel d'addition d'acide pharmaceutiquement acceptable et de l'hydroxypropylméthylcellulosephtalate dans le solvant organique, si cela se révèle souhaitable, ajouter et mélanger un ou plusieurs auxiliaires choisis parmi les excipients et des agents de désagrégation et ensuite chasser le solvant organique du mélange, par exemple, par évaporation, pour produire une matière en dispersion solide (b),
(iii) mélanger mutuellement ladite matière en dispersion solide (a), ladite matière en dispersion solide (b) et un ou plusieurs agents lubrifiants facultatifs et
(iv) comprimer le mélange ainsi obtenu pour le convertir en comprimés.

8. Procédé suivant la revendication 7, caractérisé en ce qu'il comprend en outre l'étape consistant à enrober le comprimé d'un film, à l'aide d'un agent d'enrobage.

9. Procédé suivant la revendication 7 ou la revendication 8, caractérisé en ce que le solvant organique est un alcool inférieur, un hydrocarbure halogéné, ou un mélange de ceux-ci.

## Revendications (Revendications pour l'(les) Etat(s) contractant(s) suivant(s): ES)

1. Procédé de préparation d'un comprimé à dispersion solide (DS) pharmaceutique, comprenant une matière en dispersion solide (a) qui comprend un dérivé de la 1,4-dihydropyridine, sous forme amorphe, sensiblement insoluble dans l'eau, à titre d'agent pharmaceutique actif, représenté par la formule générale (I) 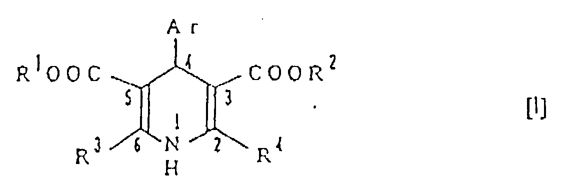 dans laquelle Ar représente un radical benzofurazannyle ou aryle substitué, R¹ représente un radical alkyle substitué ou non substitué, R² représente un radical alcényle ou un radical alkyle substitué ou non substitué, ou un groupe pipéridyle substitué, R³ et R⁴ représentent chacun indépendamment un radical alkyle substitué ou non substitué et le substituant R¹OCO- en position 5 peut être remplacé par un groupe dialkylphosphonate ou alkylènedioxyphosphoryle, ou un sel d'addition d'acide pharmaceutiquement acceptable d'un tel composé et de l'hydropropylcellulose et une matière en dispersion solide (b) qui comprend ledit dérivé de la 1,4-dihydropyridine, sous forme amorphe, ou son sel d'addition d'acide pharmaceutiquement acceptable et de l'hydroxypropylméthylcellulosephtalate, le procédé comprenant les étapes consistant à :
(i) dissoudre un dérivé de 1,4-dihydropyridine, à titre d'agent pharmaceutique actif, représenté par la formule générale (I) ci-dessus, ou un sel d'addition d'acide pharmaceutiquement acceptable d'un tel composé et de l'hydroxypropyl cellulose dans un solvant organique, si cela se révèle souhaitable, ajouter et mélanger un ou plusieurs auxiliaires choisis parmi les excipients et des agents de désagrégation et ensuite chasser le solvant organique du mélange pour produire une matière en dispersion solide (a),
(ii) dissoudre ledit dérivé de la 1,4-dihydropyridine ou son sel d'addition d'acide pharmaceutiquement acceptable et de l'hydroxypropylméthylcellulosephtalate dans le solvant organique, si cela se révèle souhaitable, ajouter et mélanger un ou plusieurs auxiliaires choisis parmi les excipients et des agents de désagrégation et ensuite chasser le solvant organique du mélange, par exemple, par évaporation, pour produire une matière en dispersion solide (b),
(iii) mélanger mutuellement ladite matière en dispersion solide (a), ladite matière en dispersion solide (b) et un ou plusieurs agents lubrifiants facultatifs et
(iv) comprimer le mélange ainsi obtenu pour le convertir en comprimés.

2. Procédé suivant la revendication 1, caractérisé en ce qu'il comprend en outre l'étape consistant à enrober le comprimé d'un film, à l'aide d'un agent d'enrobage.

3. Procédé suivant la revendication 1 ou la revendication 2, caractérisé en ce que le solvant organique est un alcool inférieur, un hydrocarbure halogéné, ou un mélange de ceux-ci.

4. Utilisation d'un mélange d'une matière en dispersion solide (a) qui comprend un dérivé de la 1,4-dihydropyridine, sous forme amorphe, sensiblement insoluble dans l'eau, sous une forme amorphe, à titre d'agent pharmaceutique actif, représenté par la formule générale (I) 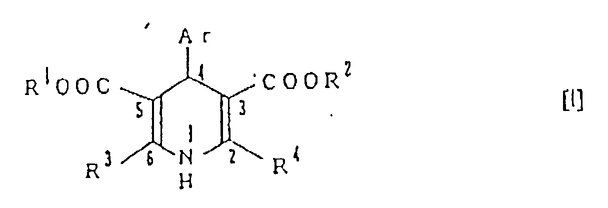 dans laquelle Ar représente un radical benzofurazannyle ou aryle substitué, R¹ représente un radical alkyle substitué ou non substitué, R² représente un radical alcényle ou un radical alkyle substitué ou non substitué, ou un groupe pipéridyle substitué, R³ et R⁴ représentent chacun indépendamment un radical alkyle substitué ou non substitué et le substituant R¹OCO- en position 5 petit être remplacé par un groupe dialkylphosphonate ou alkylénedioxyphosphoryle, ou un sel d'addition d'acide pharmaceutiquement acceptable d'un tel composé et de l'hydropropylcellulose et une matière en dispersion solide (b) qui comprend ledit dérivé de la 1,4-dihydropyridine, sous forme amorphe, ou son sel d'addition d'acide pharmaceutiquement acceptable et de l'hydroxypropylméthylcellulosephtalate, en vue de la fabrication d'un comprimé DS vasodilatateur.

5. Utilisation suivant la revendication 4, caractérisée en ce que le rapport pondéral du dérivé de la 1,4-dihydropyridine ou de son sel d'addition d'acide pharmaceutiquement acceptable à l'hydroxypropylcellulose varie de 1:4 à 1:5 et le rapport pondéral du dérivé de la 1,4-dihydropyridine ou de son sel d'addition d'acide à l'hydroxypropylméthylcellulosephtalate varie de 1:2 à 1:5.

6. Utilisation suivant la revendication 4, caractérisée en ce que le rapport de mélange de la matière en dispersion solide (a) vis-à-vis de la matière en dispersion solide (b) varie de 2,5:7,5 à 3,5:6,5 en poids.

7. Utilisation suivant la revendication 4, caractérisée en ce que les matières en dispersion solide (a) ou (b) comprennent en outre une quantité appropriée d'un ou plusieurs auxiliaires choisis parmi les excipients, des agents de désagrégation et des lubrifiants.

8. Utilisation suivant la revendication 7, caractérisée en ce que l'auxiliaire est l'aluminate-métasilicate de magnésium.

9. Utilisation suivant la revendication 8, caractérisée en ce que l'on incorpore l'aluminate-métasilicate de magnésium aux matières en dispersion solide (a) ou (b) dans le rapport pondéral du dérivé de la 1,4-dihydropyridine ou de son sel d'addition d'acide pharmaceutiquement acceptable à l'aluminate-métasilicate de magnésium de 1:11 à 1:12 ou de 1:5,5 à 1:12, respectivement.
